# EUROPEAN PATENT APPLICATION

(11) **EP 2 269 600 A1**
(43) Date of publication of application: **05.01.2011**
(21) Application number: 09290533.0
(22) Date of filing: 02.07.2009
(51) Int. Cl.: A61K 31/15, A61P 25/20, A61P 11/00

(54) **Treatment of sleep disorders using eplivanserin in COPD patients**

(71) Applicant: Sanofi-Aventis, 75013 Paris (FR)
(72) Inventor: The designation of the inventor has not yet been filed
(74) Representative: Bouron, Estelle

(57) **Abstract**

The present invention relates to eplivanserin or pharmaceutically acceptable salts or esters thereof for use in a method for treating sleep disorders in patients displaying COPD and to an article of manufacture and package, containing eplivanserin for that use.

## Description

The instant invention relates to a method of treating sleep disorders by using eplivanserin or pharmaceutically acceptable salts or esters thereof in patients displaying a chronic obstructive pulmonary disease (COPD) and who may additionally suffer from sleep disorders.

The instant invention relates to a method of providing eplivanserin or pharmaceutically acceptable salts or esters thereof.

The instant invention also relates to an article of manufacture and a package comprising eplivanserin or pharmaceutically acceptable salts or esters thereof.

It has been known for many years that neural pathways utilizing serotonin as their primary neurotransmitter play important roles in the control of sleep states. However, drugs which modulate serotoninergic transmission by, for example, stimulating or blocking receptors for this transmitter, have never found clinical use in the treatment of insomnia (Ann Pharm Fr 2007, vol. 65 pp. 268-274). This may soon change as several agents with selective activity as antagonists or inverse agonists at the 5HT_{2A} subtype of serotonin receptors are currently being tested in patients with sleep disorders. 5HT_{2A} antagonists do not show pharmacological effects similar to traditional sedative hypnotic agents. However, they do modify sleep architecture by selectively increasing slow wave sleep *(*Neuropsychopharmacology (1999); vol.21, pp. 455-466).

The compound (1Z, 2E)-1-(2-fluorophenyl)-3-(4-hydroxyphenyl)-prop-2-en-1-one-O-(2-dimethylaminoethyl)oxime, is hereafter referenced as eplivanserin or pharmaceutically acceptable salts or esters thereof. Documents EP 373998 and US 5166416 claim compounds having a generic scope encompassing eplivanserin or pharmaceutically acceptable salts or esters thereof and also specifically claimed eplivanserin or pharmaceutically acceptable salts or esters thereof, and disclosed its use as a platelet anti-aggregant or a psychotropic agent.

See also US 6143792 and EP 1014960 (sleep apnea) and US 6576970 and EP 11140955 (snoring and upper airway resistance syndrome).

Eplivanserin or pharmaceutically acceptable salts or esters thereof, in particular hemifumarate salt, is an antagonist of 5HT_{2A} receptors (Journal of Pharmacological Experiment in Therapeutics, (1992), vol. 262 (2), pp. 759-68). Eplivanserin is well absorbed (>70%). Conventional dosage, between 1 and 10 mg, leads to a maximal plasma concentration that is reached between 2 and 6 hours; the half-life time of eplivanserin is relatively long, with an average value of 50 hours. Eplivanserin or pharmaceutically acceptable salts or esters thereof is also known to enhance slow wave sleep (SWS) (Neuropsychopharmacology (1999), vol.21 (3), pp. 455-466).

It has been shown that eplivanserin or pharmaceutically acceptable salts or esters thereof, by enhancing SWS, may significantly improve sleep maintenance and quality of sleep (QoS) without inducing next-day sedation and rebound or withdrawal symptoms after treatment discontinuation in patients with chronic insomnia and sleep maintenance difficulties.

Chronic obstructive pulmonary disease (COPD) encompasses a range of respiratory diseases including bronchitis, emphysema and others. This disease is defined as a progressive limitation of functional airflow that is not fully reversible with inhaled bronchodilatators (ATS S. Am. J. Respir Crit Care Med 1995 152: S78-S119). The disease is progressive and chronic, requiring long-term treatment to improve quality of life in affected patients. Several comorbidities accompany COPD including unexplained weight loss, cardiovascular disease, peripheral muscle weakness, cognitive impairment, depression, anxiety and sleep disorders (Roth T. et al. Sleep medicine 2009, 10: 19-25).
COPD patients are more likely to have difficulties falling and staying asleep and have increased sleepiness during the day. Moreover, the quality of sleep is significantly compromised in many patients with COPD. A reduced sleep quality is associated with daytime consequences like fatigue, psychiatric problems and an impaired quality of life. Arousals from sleep are more likely in these patients due to chronic coughing and nocturnal wheezing and also nocturnal oxygen desaturation (Gay PC. 2004 Respir Care 49(1): 39-51). Over 50% of COPD patients report long sleep latency, frequent arousals during the night and/or general insomnia (George CF. Sleep 200; 23: S31-S35). Analysis of a large COPD database revealed that 21.4% of the listed COPD patients were diagnosed with and were treated for insomnia as compared to only 7.2% non-COPD patients (Vallarino CR. 2005 Value Health 8(3): 322).

Sleep induces physiological alterations in respiratory functions, which can become pathologic and may provoke or worsen hypoxemia and hypercapnia in COPD. Dyspnea, cough and excessive mucus production should be optimised to minimise causes for sleep disturbance. Pharmacological therapy may be helpful; however some sedatives like benzodiazepines and non-benzodiazepine-receptor agonists may produce serious adverse respiratory effects during sleep (Stege G. et al. 2008 Respiratory Medicine 102, 801-814; Roth T. 2009 Sleep Medicine 10: 19-25)). Therefore their use, if allowed, should be as short as possible.

The benzodiazepines (BZ) traditionally prescribed for insomnia include those indicated specifically for insomnia, namely, temazepam, triazolam, flurazepam, estazolam, and quazepam among others. However, several reports describe adverse pulmonary events associated with benzodiazepine usage in COPD patients. Some trials have reported adverse effects on pulmonary function in patients with COPD (Jolly E. 1996 Medicina 56: 472-478; Cohn MA. 1992 Drug Safety 7(2): 152-158; Beaupre A. et al. 1988 Respiration 54(4): 235-40). Thus benzodiazepines depress respiratory functions that participate in maintaining homeostasis of arterial blood gases during sleep.

A newer generation of BZ receptor agonists (BZRAs), also known as non-benzodiazepines, has a higher selectivity for BZ receptor subtypes expressed predominantly in neurons of the CNS. These drugs have little to no affinity for the other GABA_{A} receptor subtypes, and have fewer adverse effects on respiratory function than do the traditional benzodiazepines. Furthermore, these drugs specifically target the receptor believed to mediate the sedation effects of the BZRAs.

The non-benzodiazepine BZRAs have similar effects on sleep latency and efficiency as traditional benzodiazepines. Several clinical studies have been conducted to test the effects of these drugs on pulmonary function. Unlike benzodiazepines, these BZRAs have been found to have no significant effect on ventilatory drive and central control of breathing in normal subjects or in patients with mild to moderate COPD (A. Beaupre et al. Respiration 54 (4) (1988), pp. 235-240; P.J. Ranlov et al., Sleep 10 (Suppl. 1) (1987), pp. 40-47; C. Girault, J et al., Chest 110 (5) (1996), pp. 1203-1211).

But although non-benzodiazepines have fewer apparent adverse effects on pulmonary function, the potential for adverse effects on cognitive function may complicate treatment of insomnia in COPD patients, many of whom suffer from cognitive impairment or are elderly patients at risk for fall-related fractures (R. Antonelli-Incalzi, et al., Dement Geriatr Cogn Disord 23(4) (2007), pp. 264-270).

Altogether, these observed effects suggest that there are significant safety concerns associated with the use of hypnotics in the COPD population and a real need for a safe insomnia treatment in this population.

Clinical trials with eplivanserin have shown that it can be safely administered to patients displaying COPD, without causing an impairment of respiratory function, in comparison with placebo.

One method according to the invention is performed by administering a therapeutic amount of eplivanserin or pharmaceutically acceptable salts or esters thereof in patients patients displaying COPD and who may additionally suffer from sleep disorders.

The invention thus concerns eplivanserin or pharmaceutically acceptable salts or esters thereof for the treatment of sleep disorders in patients displaying COPD, and in patients with COPD and who may additionally suffer from sleep disorders, a therapeutic amount of eplivanserin or pharmaceutically acceptable salts or esters thereof being administered.

The invention thus concerns the use of eplivanserin or pharmaceutically acceptable salts or esters thereof for the preparation of a medicament for the treatment of patients displaying COPD and who may suffer from sleep disorders. In some embodiments, a pharmaceutically acceptable salt of eplivanserin is hemifumarate.
In some embodiments, sleep disorders are insomnia.
In some embodiments, sleep disorders are chronic insomnia.
In some embodiments, sleep disorders are insomnia characterized by sleep maintenance difficulties.
In some embodiments, sleep disorders are insomnia including nocturnal awakenings, usually for short-term duration.
In some embodiments, sleep disorders are insomnia including nocturnal awakenings or early awakenings, usually for short-term duration.
In some embodiments, sleep disorders are chronic insomnia characterized by difficulties with sleep maintenance.
In some embodiments, COPD is mild or moderate COPD.

Another method according to the invention is performed by providing eplivanserin or pharmaceutically acceptable salts or esters thereof, wherein said eplivanserin or pharmaceutically acceptable salts or esters thereof is provided along with information indicating that eplivanserin can be safely administered to patients displaying COPD and who may suffer from sleep disorders.

In some embodiments, the information comprises printed matter that advises that eplivanserin or pharmaceutically acceptable salts or esters can be safely and effectively administered to patients displaying COPD and who may suffer from sleep disorders. In some embodiments, said printed matter is a label.

The invention also relates to an article of manufacture comprising:
a) a packaging material;
b) eplivanserin or pharmaceutically acceptable salts or esters thereof or, and
c) a label or package insert contained within the packaging material indicating that eplivanserin can safely and effectively be used in patients displaying COPD.
In some embodiments, a pharmaceutically acceptable salt of eplivanserin is hemifumarate.

The invention also relates to a package comprising eplivanserin or pharmaceutically acceptable salts or esters thereof and a label, said label comprising a printed statement which informs a prospective user that eplivanserin can be safely and effectively used in patients displaying COPD and who may suffer from sleep disorders

In some embodiments of all the methods according to the invention, a pharmaceutically acceptable salt of eplivanserin is hemifumarate.
In some embodiments of all the methods according to the invention, sleep disorders are insomnia.
In some embodiments of all the methods according to the invention, sleep disorders are chronic insomnia.
In some embodiments of all the methods according to the invention, sleep disorders are insomnia characterized by sleep maintenance difficulties.
In some embodiments of all the methods according to the invention, sleep disorders are insomnia including nocturnal awakenings, usually for short-term duration.
In some embodiments of all the methods according to the invention, sleep disorders are insomnia including nocturnal awakenings or early awakenings, usually for short-term duration. In some embodiments of all the methods according to the invention, sleep disorders are chronic insomnia characterized by difficulties with sleep maintenance.
In some embodiments, COPD is mild or moderate COPD.

The term "treating" or "treat" refers to either preventing, providing symptomatic relief, or curing the patient's disease, disorder or condition.
In the context of the present patent application, the term "sleep disorders" especially means, insomnia, primary insomnia, sleep maintenance insomnia, insomnia associated with a mental disease, comorbid insomnia, i.e. insomnia associated with sleep apnea, pain, diabetes, depression, anxiety.
The term "insomnia" (Diagnostic and Statistical Manual of Mental Disorders, fourth edition criteria) includes difficulty in maintaining sleep, with multiple nocturnal awakenings.

The term "administering" comprises administration via any appropriate unitary dosage forms for eplivanserin or pharmaceutically acceptable salts or esters thereof such as oral forms, such as tablets, hard or soft gelatin capsules, powders, granules and oral solutions or suspensions, as well as the sublingual, buccal, intratracheal, intraocular, intranasal forms, the forms adapted to inhalation, topical, transdermal, sub-cutaneous, intramuscular or intravenous delivery, the rectal forms and the implants. For the topical application, eplivanserin or pharmaceutically acceptable salts or esters thereof may be used as creams, gels, ointments or lotions.
For eplivanserin or pharmaceutically acceptable salts or esters thereof, tablets can be a preferred mode of administration.

The term "therapeutic amount" means enough of the compound which becomes available through the appropriate route of administration to treat the patient for the disorder, the condition or the disease.
For eplivanserin or pharmaceutically acceptable salts or esters thereof, a therapeutic amount is 5 mg once a day. In some embodiments, the administration can be done either in the morning or in the evening, just before a sleep period, or at any time of the day.
However in specific cases, different dosages may be appropriate; these dosages are comprised within the scope of the present invention. According to usual practice, the dosage suitable to each patient is determined by the physician according to, f or example, the administration route, the weight and response of the patient.

The term "providing" includes selling, distributing, shipping, offering for sell, importing etc.

The instant invention is illustrated by the clinical data below.

### Example 1:

The effects of eplivanserin or pharmaceutically acceptable salts or esters thereof on sleep architecture and sleep parameters in patients with sleep disorders were examined.

The Phase 3 program of development of eplivanserin in the treatment of patients with chronic (primary) insomnia characterized by sleep maintenance difficulties consisted of 3 randomized, double-blind (DB), placebo-controlled, efficacy and safety studies designed to assess the efficacy of eplivanserin on sleep maintenance either using polysomnography (PSG) recordings (6-week study EFC6220) or patient-reported outcome (12-week studies LTE6217 and LTE6262) collected daily on sleep questionnaires.
They were all double-blind (DB), randomized, placebo-controlled studies. In all studies, treatment period was preceded by a one week, placebo run-in period, designed to check selection criteria for insomnia based on patient reported sleep parameters collected on the daily sleep questionnaires (Studies LTE6217, and LTE6262) or based on PSG recordings during two screening nights in a sleep laboratory (Study EFC6220). Treatment period was followed by a placebo run-out period of 2 weeks designed to assess the effect of abrupt treatment discontinuation.
Study EFC6220 was designed to measure the pharmacodynamic activity of eplivanserin on sleep maintenance using PSG recordings in a sleep laboratory. Polysomnograms were recorded in standard conditions during 8 hours in bed.
Studies LTE6217 and LTE6262 measured the efficacy of eplivanserin in real life conditions of use at home by collecting daily sleep parameters reported by patients through an Interactive Voice Response System (IVRS).

The results of Study EFC6220 confirmed the expected clinical activity of eplivanserin on sleep maintenance by decreasing PSG-WASO at Week 3 and Week 6, but the difference with placebo did not reach statistical significance at Week 6. However, eplivanserin 5 mg was shown to consistently decrease PSG-NAW at both time points (LS mean =-1.74 and -1.82, p<0.0001 at Week 3 and Week 6 respectively), associated with a decrease of pr-NAW. Eplivanserin decreased the PSG-NAW mainly from H1 up to H6 compared with placebo. The decrease of wakefulness was not associated with an increase of the final awakening. The duration of the remaining awakenings was not increased. In addition, eplivanserin decreased the number of brief awakenings (<1 min) as compared to placebo confirming its effect on sleep consolidation.
In addition, the QoS was improved and a majority of patients declared that eplivanserin "helped them sleep" (PGI-item 1) and "increased their duration of sleep" (PGI-item 3). Comparable results were observed in the elderly patients (≥65 years).
Eplivanserin 5 mg did not affect sleep onset as shown by the results of PSG-LPS and pr-SOL.
The analysis of sleep architecture showed an increased percentage of time spent in sleep Stages 3&4 (SWS or deep sleep) with eplivanserin 5 mg/day compared with placebo, associated mainly with a decrease of Stage 1 (a stage close to wakefulness). The per hour analysis of SWS showed that eplivanserin increased SWS from H2 to H6 in comparison with placebo with a maximum at H3 while respecting the biological structure of sleep. The increase of SWS/WASO + Stage 1 index observed with eplivanserin also confirmed the decrease of sleep fragmentation and improved quality of sleep with eplivanserin. REM sleep was not affected by eplivanserin.

Eplivanserin 5 mg/day improved sleep maintenance by consistently and significantly decreasing pr-WASO (studies LTE6217 and LTE6262), reducing the mean pr-NAW and increasing the pr-TST after 6 and 12 weeks of treatment. Eplivanserin also improved the Quality of Sleep as well as the Refreshing Quality of Sleep after 6 and 12 weeks of treatment, in patients with insomnia characterized by sleep maintenance difficulties. Eplivanserin did not affect sleep onset. Patients reported that they perceived eplivanserin 5 mg/day as an "aid to sleep" (PGI-item 1) and as "increasing duration of sleep" (PGI-item 3) in comparison with placebo, after 6 and 12 weeks of treatment. In addition, a positive effect of eplivanserin compared with placebo was observed regarding the improvement of activities related to work and hobby activities after 12 weeks of treatment in Study LTE6262 and the meta-analysis of the 2 long-term studies (LTE6262 and LTE6217).
Results from the safety analyses on potential for residual effects not only showed that eplivanserin was devoid of next-day impairment but that eplivanserin was reported by insomniac patients to have a significantly favorable effect on "sleepiness in the morning" and "ability to concentrate" as compared with placebo.
Results in the elderly sub-population were consistent with those of the global population.

The effect of eplivanserin 5 mg/day on sleep maintenance was observed from Day 1 and persisted up to 1 year of treatment with a stable decreased of mean change from baseline for pr-WASO and no apparent dose increase. These results indicate that eplivanserin has an early onset of action and a maintained efficacy potential for the long-term treatment of chronic insomnia.

These results showed that eplivanserin promoted sleep maintenance by enhancing the deep component of sleep (SWS) and by decreasing the "hyper-arousal" state of insomniac patients and sleep fragmentation offering patients a more consolidated sleep. The analysis of sleep architecture also allowed the demonstration that, unlike benzodiazepine hypnotics, eplivanserin preserved and even restored the physiological structure of sleep in insomniac patients. Finally, patients perceived the decrease in the WASO and NAW and reported that eplivanserin improved their sleep quality and refreshing sleep.

### Example 2: COPD study

The primary objective of this phase I study was to evaluate the pharmacodynamic (PD) effects of eplivanserin 15 mg single dose versus placebo (P) on respiratory function measured by mean overnight oxygen saturation (SaO2) in patients with mild to moderate COPD.
Recorded PD parameters were:
- Pulse oximetry continuously recorded overnight, with 02 saturation in %,
- Pulmonary function tests (PFTs): spirometry [measuring forced vital capacity (FVC), forced expiratory volume (FEV), peak exploratory flow rate (PEFR), forced exploratory flow (FEF)] and plethysmography [evaluating airways resistance and conductance],
- Capillary blood gases,
- PSG with apnea /hypopnea episodes, sleep global sleep parameters and sleep architecture,
- Leeds Sleep Evaluation Questionnaire (LSEQ) and Bond & Lader VAS. Safety and pharmacokinetic parameters were also assessed.

The study was a single-centre, double-blind, randomized, 2-way crossover, placebo-controlled crossover study with 14 day washout: 15 mg single-dose of eplivanserin (E) vs. placebo (P).
Included patients were displaying mild to moderate chronic obstructive pulmonary disease (COPD).
The primary end-point was the pulmonary function (02 saturation over the entire night). 28 evaluable patients have been evaluated (COPD severity (FEV1) 50% mild / 50% moderate; Gender: 46.4% M, 53.6% F)

### Results:

**Table 1: oxygen saturation during the night**

| **Treatment difference estimates for SaO2 (%) during time in bed** | | | | |
|---|---|---|---|---|
| | **Comparison** | **LS mean** | **P-value** | **95% Cl** |
| SaO2 (%) during Time in bed | Eplivanserin 15 mg - Placebo | -0.30 | 0.2499 | [-0.83;0.23] |

| | | | | |
|---|---|---|---|---|
| LS mean = Least Square mean | | | | |

Mean oxygen saturation over the entire night assessed by finger pulse oximetry did not show any significant difference between eplivanserin 15 mg (mean ± SD, 93.4 % ± 1.66) and P. (mean ± SD, 93.8 % ± 1.29)
A single dose of eplivanserin 15 mg had no effect on SaO2 (%) vs placebo during any the sleep stages (stage 1, 2, SWS, REM, wake).

**Table 2: spirometry parameters**

| **Treatment difference estimates for spirometry parameters** | | | | |
|---|---|---|---|---|
| | **Comparison** | **LS mean** | **P-value** | **95% Cl** |
| Forced expiratory volume (liters) | Eplivanserin 15 mg - Placebo | -0.03 | 0.5289 | [-0.14;0.07] |
| Forced vital capacity (liters) | Eplivanserin 15 mg - Placebo | -0.12 | 0.1411 | [-0.28;0.04] |
| Peak expiratory flow (L/S) | Eplivanserin 15 mg - Placebo | -0.05 | 0.6683 | [-0,26;0.17] |

| | | | | |
|---|---|---|---|---|
| LS mean = Least Square mean There was no difference in SaO2 (%) after eplivanserin 15mg or Placebo, in each patients' subgroup (mild, moderate) | | | | |

**Table 3: plethysmography parameters**

| **Treatment difference estimates for plethysmography parameters** | | | | |
|---|---|---|---|---|
| | **Comparison** | **LS mean** | **P-value** | **95% Cl** |
| Airways resistance (KPa * s/l) | Eplivanserin 15 mg - Placebo | 0.02 | 0.4880 | [-0.04;0.08] |
| Airways conductance (1/KPa * s) | Eplivanserin 15 mg - Placebo | -0.01 | 0.6642 | [-0.05;0.03] |

| | | | | |
|---|---|---|---|---|
| LS mean = Least Square mean | | | | |

There were no difference in airways resistance and conductance in each patient's subgroup (mild, moderate).

Other PD assessments have shown that:
- Capil lary blood gases were minimally or unaffected (pH, oxygen partial pressure)
- PSG results were consistent with those observed on eplivanserin in previous PSG studies, in both healthy subjects and insomniac patients: reduction of the AHI, reduction of WASO, increase in TST, reduction of the number of awakenings after sleep onset and no change in LPS
- LSEQ and Bond & Lader VAS: post-treatment scores vs baseline were minimally or unaffected
- The re was no safety concern
AHI: apnea hypopnea index
WASO: waketime duration after sleep onset
TST: total sleep time
LPS: latency to persistent sleep
LSEQ: Leeds Sleep Evaluation Questionnaire

### Conclusions:

After an oral single dose administration of eplivanserin 15 mg in patients with mild or moderate COPD, no impairment of respiratory function was observed in comparison with placebo, as measured by overnight mean oxygen saturation (SaO2%), spirometry and body plethysmography.
The effects of eplivanserin on polysomnographic parameters were consistent with those previously described for this drug, and posttreatment LSEQ and Bond and Lader VAS scores showed little or no changes compared to baseline.
Similar results were observed between mild and moderate COPD, based on descriptive statistics.
Eplivanserin 15 mg in single dose administration was well tolerated in this population of patients with mild or moderate COPD. In particular, no worsening of their respiratory condition was noted. Eplivanserin can thus be safely and effectively administered in patients displaying COPD and who may additionally suffer from sleep disorders.

## Claims

**1.** Eplivanserin or pharmaceutically acceptable salts or esters thereof for the treatment of sleep disorders in patients displaying a chronic obstructive disease (COPD), a therapeutic amount of eplivanserin or pharmaceutically acceptable salts or esters thereof being administered.

**2.** Eplivanserin or pharmaceutically acceptable salts or esters thereof for the treatment of sleep disorders in patients displaying a chronic obstructive disease (COPD) and who may additionally suffer from sleep disorders, a therapeutic amount of eplivanserin or pharmaceutically acceptable salts or esters thereof being administered.

**3.** Eplivanserin according to claim 1, wherein a pharmaceutically acceptable salt of eplivanserin is hemifumarate.

**4.** Eplivanserin according to claim 1 or 2, wherein sleep disorders are insomnia.

**5.** Eplivanserin according to claim 1 or 2, wherein sleep disorders are chronic insomnia.

**6.** Eplivanserin according to anyone of claims 1 to 4, wherein sleep disorders are insomnia **characterized by** difficulties with sleep maintenance.

**8.** Eplivanserin according to anyone of claims 1 to 5, wherein sleep disorders are chronic insomnia **characterized by** difficulties with sleep maintenance.

**9.** Eplivanserin according to anyone of claims 1 to 7, where COPD is mild to moderate COPD.

**10.** Eplivanserin for a safe and effective use in patients displaying COPD.

**11.** Method of providing eplivanserin or pharmaceutically acceptable salts or esters, wherein said eplivanserin or pharmaceutically acceptable salts or esters thereof is provided along with information indicating that eplivanserin or pharmaceutically acceptable salts or esters thereof can be safely administered to patients displaying COPD and who may suffer from sleep disorders.

**12.** Method according to claim 9, wherein a pharmaceutically acceptable salt of eplivanserin is hemifumarate.

**13.** Method according to claim 10, wherein sleep disorders are insomnia.

**14.** Method according to claim 9 or 10, wherein sleep disorders are chronic insomnia.

**15.** Method according to anyone of claims 9 to 12, wherein sleep disorders are insomnia **characterized by** difficulties with sleep maintenance.

**16.** Method according to anyone of claims 9 to 13, wherein sleep disorders are chronic insomnia **characterized by** difficulties with sleep maintenance.

**17.** Method according to anyone of claims 9 to 14, where the COPD is mild to moderate COPD.

**18.** An article of manufacture comprising
a) a packaging material;
b) eplivanserin or pharmaceutically acceptable salts or esters thereof or, and
c) a label or package insert contained within the packaging material indicating that eplivanserin or pharmaceutically acceptable salts or esters thereof can be safely administered to patients displaying COPD and who may suffer from sleep disorders.

**19.** An article according to claim 16, wherein a pharmaceutically acceptable salt of eplivanserin is hemifumarate.

**20.** A package comprising eplivanserin or pharmaceutically acceptable salts or esters thereof and a label, said label comprising a printed statement which informs a prospective user that eplivanserin or pharmaceutically acceptable salts or esters thereof can be safely administered to patients displaying COPD and who may suffer from sleep disorders.

**21.** A package according to claim 18, wherein a pharmaceutically acceptable salt of eplivanserin is hemifumarate.
